# EUROPEAN PATENT SPECIFICATION

(11) **EP 3 628 253 B1**
(45) Date of publication and mention of the grant of the patent: **03.11.2021**
(21) Application number: 18908274.6
(22) Date of filing: 23.08.2018
(51) Int. Cl.: A61B 18/12, A61B 18/14, A61B 90/30, A61B 17/00

(54) **HIGH FREQUENCY-CURRENT SCALPEL WITH LOCKABLE TELESCOPIC LENGTH HAVING ILLUMINATION AND SMOKE ASPIRATION FUNCTIONS**
RAUCHABSORBIERENDES HOCHFREQUENTES CHIRURGISCHES MESSER MIT BELEUCHTUNGSFUNKTION UND FÄHIGKEIT ZUR VERRIEGELUNG DER TELESKOPISCHEN LÄNGE
SCALPEL À COURANT HAUTE FRÉQUENCE À LONGUEUR TÉLESCOPIQUE VERROUILLABLE PRÉSENTANT DES FONCTIONS D'ÉCLAIRAGE ET D'ASPIRATION DE FUMÉE

(30) Priority: 13.08.2018 CN 201810914786
(43) Date of publication of application: 01.04.2020
(73) Proprietor: Zhejiang Medstar Technology Co., Ltd, Jiaxing City, Zhejiang 314050 (CN)
(72) Inventor: HUANG, HaiJun, Jiaxing Zhejiang 314050 (CN); CHAO, Yu, Jiaxing Zhejiang 314050 (CN); WANG, QingHua, Jiaxing Zhejiang 314050 (CN); YIN, YanHui, Jiaxing Zhejiang 314050 (CN); LIU, TianGuo, Jiaxing Zhejiang 314050 (CN)
(74) Representative: Ipside
(86) International application number: PCT/CN2018/102017
(87) International publication number: WO 2020/034246

(56) References cited:
- WO-A1-2017/178286
- CA-A1- 2 488 877
- CN-A- 104 434 301
- CN-A- 104 473 690
- CN-A- 104 546 116
- CN-A- 106 691 582
- CN-U- 201 510 348
- US-A1- 2012 283 728
- US-A1- 2016 157 920
- US-A1- 2018 110 582

## Description

### TECHNICAL FIELD OF THE INVENTION

The present invention relates to the technical field of medical instruments, and more particularly, to a smoke-absorbing high-frequency surgical knife with illumination function and capable of locking the telescopic length.

### BACKGROUND OF THE INVENTION

Medical instruments represented by surgical knives are widely used. During surgery, the separation, cutting, stitching and bleeding-stop of human tissues (e.g. skin) must be achieved accurately and quickly. The existing surgical knives mainly comprise a high-frequency electric knife, a laser electric knife, a microwave surgical knife, an ultrasonic surgical knife and a gamma knife, etc. Normally, each of the knives has a specific application area, but also has own technical defects at different degrees.

US 2012/2012/283728 A1 discloses an electrosurgery pencil comprising: a handpiece having an open first end; an electrode contained within at least a portion of the handpiece wherein at least a portion of the electrode extends beyond the open first end of the handpiece; at least one electrical contact in communication with said electrode for enabling at least one of cutting and coagulation during a medical procedure; and at least one lighting element located near the open first end of the handpiece.

In order to achieve an illuminating effect, the existing surgical knives usually adopt an external illumination. In other words, one or more LED lamp beads are installed outside the surgical knife handle near the position where the electrode is located, thereby projecting the light rays onto the surgical spot. However, the aforesaid technical solution has the following shortcomings:
First, no matter how many LED lamp beads are installed outside the surgical knife handle, for the LED lamp beads are scattered, there's still a shadow existing in the illumination environment formed by the overlapped light rays;
Second, when the illuminating brightness needs to be improved, more LED lamp beads are required to be installed, directly increasing the space occupation and seriously affecting the field of vision during surgery;
Third, for the LED lamp beads are fixedly arranged on the surgical knife handle, they cannot stretch and retract synchronously with the electrode. The LED lamp beads are far away from the position where the electrode can effectively act. When a long electrode is adopted, or when the electrode extends out, the illuminating effect can plummet, resulting in a more severe shadow;
Fourth, it's impossible for the existing surgical knives to simultaneously be shadow-free, illuminable, smoke-absorbing and telescopic. Common surgical knives normally possess two functions selected from shadow-free, smoke-absorbing and telescopic.

### SUMMARY OF THE INVENTION

The purpose of the present invention is to solve the shortcomings in the prior art by providing a smoke-absorbing high-frequency surgical knife with illumination function and capable of locking the telescopic length.

To achieve the above purpose, the present invention adopts the following technical solution:
A smoke-absorbing high-frequency surgical knife with illumination function and capable of locking a telescopic length comprising a surgical knife upper shell and a surgical knife main body, wherein the surgical knife upper shell is located above the surgical knife main body, and is embedded with the surgical knife main body; a first accommodating chamber is arranged between the surgical knife upper shell and the surgical knife main body, and two double-sided PCBs are arranged in the first accommodating chamber, wherein a circuit on one side of one double-sided PCB is used for switching on/off a high-frequency current of the high-frequency electrode according to second or third control signals triggered by the user, the circuit on the other side of the aforesaid double-sided PCB is adapted to be used for turning on/off an LED light source (different from SMD LED lamp beads and used for environmental illumination), and the other double-sided PCB is adapted to be used for turning on/off the SMD LED lamp beads according to a first control signal triggered by the user; a second accommodating chamber is arranged in the surgical knife main body; the smoke-absorbing high-frequency surgical knife with illumination function and capable of locking the telescopic length further comprises a smoke-absorbing tube, a locking mechanism, a light-emitting element, and a high-frequency electrode, wherein a portion of the smoke-absorbing tube is located in the second accommodating chamber, the portion of the smoke-absorbing tube is provided with a slide groove for smoke-absorbing, and the SMD LED lamp beads are arranged in an annular shape in an illuminating component; the other portion of the smoke-absorbing tube is located outside the surgical knife main body, and the portion of the smoke-absorbing tube is internally connected with the locking mechanism; the light-emitting element is internally connected with the smoke-absorbing tube, and an inner surface of the smoke-absorbing tube integrally extends to form an electrode fixing seat; the high-frequency electrode is coaxially arranged with the light-emitting element; one end of the high-frequency electrode is located on the outer side of the light-emitting element, and is adapted to keep the absolute distance from the light-emitting element unchanged; the other end of the high-frequency electrode passes through the light-emitting element and is internally connected to the electrode fixing seat; thus, when the smoke-absorbing tube stretches and retracts relative to the axial direction of the surgical knife main body, the high-frequency electrode, the light-emitting element and the SMD LED lamp beads synchronously stretch and retract relative to the axial direction of the surgical knife main body; wherein the power supply line of the SMD LED lamp beads and the high-frequency line of the high-frequency electrode are mutually independent, further enhancing the stability and safety of the smoke-absorbing high-frequency surgical knife with illumination function and capable of locking the telescopic length.

In another aspect of the present invention, the SMD LED lamp beads are wrapped by a light-transmitting plastic material, thereby achieving a water-resistant effect.

In another aspect of the present invention, the smoke-absorbing high-frequency surgical knife with illumination function and capable of locking the telescopic length further comprises a buckle and an annular sealing ring. The locking mechanism comprises an outer locking mechanism, and the annular sealing ring is internally arranged in the outer locking mechanism. The buckle is provided with a buckle lower surface protruding portion. One end of the buckle is internally connected with the outer locking mechanism, and the other end of the buckle is embedded into the surgical knife main body. The buckle lower surface protruding portion of the buckle is embedded into the slide groove for smoke-absorbing tube.

In another aspect of the present invention, the locking mechanism comprises an inner locking mechanism, and the inner locking mechanism comprises a conductive tube. The smoke-absorbing tube is internally connected with a conductive tube, and one end of the smoke-absorbing tube is sleeved with an eccentric tensioning ring. The end portion of the smoke-absorbing tube sleeved with the eccentric tensioning ring is provided with an eccentric groove annularly arranged along the outer surface of the end portion of the smoke-absorbing tube. The eccentric tensioning ring is partially and externally connected with the eccentric groove of the smoke-absorbing tube. The distance between the outer periphery of the eccentric groove and the inner surface of the conductive special-shaped tube varies periodically in the radial direction of the smoke-absorbing tube.

In another aspect of the present invention, the middle of the upper surface of the buckle is provided with a buckle upper surface protruding portion. A plurality of grooves with different depths are formed in one side of the buckle upper surface protruding portion arranged on the upper surface of the buckle, and a reverse slope is provided on the other side of the buckle upper surface protruding portion arranged on the upper surface of the buckle. The tail end of the buckle that is provided with the plurality of grooves with different depths abuts against the annular sealing ring. The inner surface of the outer locking mechanism is provided with a plurality of convex rings with different heights, and each of the grooves with different depths on the upper surface of the buckle respectively interacts with each of the convex rings with different heights on the inner surface of the outer locking mechanism.

In another aspect of the present invention, the smoke-absorbing high-frequency surgical knife with illumination function and capable of locking the telescopic length further comprises a fixing seat of the conductive spring of the smoke-absorbing tube and a high-frequency conductive spring. One end of the smoke-absorbing tube is internally connected with the fixing seat of the conductive spring of the smoke-absorbing tube . One end of the high-frequency conductive spring is internally connected with the fixing seat of the smoke- conductive spring of the absorbing tube , and the other end of the high-frequency conductive spring is internally connected with the high-frequency conductive copper groove.

In another aspect of the present invention, the smoke-absorbing high-frequency surgical knife with illumination function and capable of locking the telescopic length further comprises an anti-slip sleeve, and the surgical knife main body is covered by the anti-slip sleeve.

In another aspect of the present invention, the smoke-absorbing high-frequency surgical knife with illumination function and capable of locking the telescopic length further comprises a bent joint, a hose and a current carrying wire, wherein one side of the bent joint is simultaneously connected to the surgical knife upper shell and the surgical knife main body in a sleeved mode, and the other side of the bent joint is internally connected with the hose. The current carrying wire extends into the first accommodating chamber formed between the surgical knife upper shell and the surgical knife main body through the bent joint.

In another aspect of the present invention, the smoke-absorbing high-frequency surgical knife with illumination function and capable of locking the telescopic length further comprises an illuminating button, an electric coagulation button and an electric cutting button, wherein the illuminating button is used for inputting the first control signal, the electric coagulation button is used for inputting the second control signal, and the electric cutting button is adapted to be used for inputting the third control signal. The surgical knife upper shell is provided with a first positioning hole, a second positioning hole and a third positioning hole, wherein the illuminating button, the electric coagulation button and the electric cutting button are respectively embedded into the first positioning hole, the second positioning hole and the third positioning hole.

Compared with the prior art, the present invention has the following advantages:
The present invention adopts an internal illumination mode. When the smoke-absorbing tube stretches and retracts, the high-frequency electrode, the light-emitting element, and the SMD LED lamp beads synchronously stretch and retract along with the electrode. The light-emitting element is close to the position where the high-frequency electrode effectively acts, and keeps the absolute distance unchanged, achieving a stable illumination intensity at the acting position of the high-frequency electrode. The illuminating component, namely, the SMD LED lamp beads, annularly and uniformly emit light, ensuring the surgical spot is shadow-free.
The locking degree of the locking mechanism can be finely adjusted so that the locking function can be achieved at any position.

### BRIEF DESCRIPTION OF THE DRAWINGS

Figure 1 is a three-dimensional structural diagram of the preferred embodiment of the present invention;
Figure 2 is an explosive view of the preferred embodiment of the present invention;
Figure 3 is an explosive view of the preferred embodiment from another angle of the present invention;
Figure 4 is a three-dimensional structural diagram of the first modification of the preferred embodiment of the present invention;
Figure 5 is an explosive view of the first modification of the preferred embodiment of the present invention;
Figure 6 is a three-dimensional structural diagram of the second modification of the preferred embodiment of the present invention;
Figure 7 is an explosive view of the second modification of the preferred embodiment of the present invention;

### Marking Instructions of the Drawings:

10-Surgical Knife Upper Shell, 11- The First Positioning Hole, 12-The Second Positioning Hole, 13-The Third Positioning Hole, 14-Illuminating Button, 15-Electric Coagulation Button, 16-Electric Cutting Button, 20-Surgical Knife Main Body, 30-Double-sided PCB, 40-High-frequency Electrode, 50-Smoke-absorbing Tube, 51-Smoke-absorbing Tube Upper Portion, 52-Smoke-absorbing Tube Lower Portion, 53-Slide Groove for Smoke-absorbing Tube, 54-High-frequency Conductive Copper Groove, 55-Buckle, 56-Annular Sealing Ring, 57- Fixing Seat of the Conductive Spring of the Smoke-absorbing Tube , 60-Locking Mechanism, 70-Light-emitting Element, 81-Bent Joint, 82-Hose, 83-Current Carrying Wire, 84-Anti-slip Sleeve, 90-Illuminating Component, 91-Illuminating Ring, 92-Illuminating Body, 93-SOC Part, 94-Conductive Special-shaped Tube, 95-Eccentric Tensioning Ring, 96-Eccentric Ring.

### DETAILED DESCRIPTION OF THE INVENTION

The present invention discloses a smoke-absorbing high-frequency surgical knife with illumination function and capable of locking the telescopic length. Preferred embodiments are combined hereinafter to elaborate the specific implementation way of the present invention.

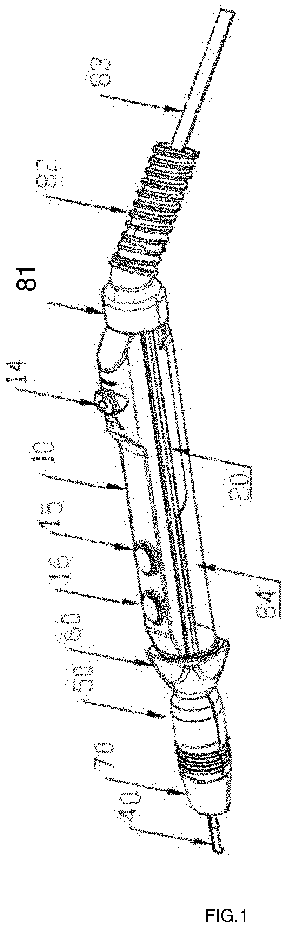

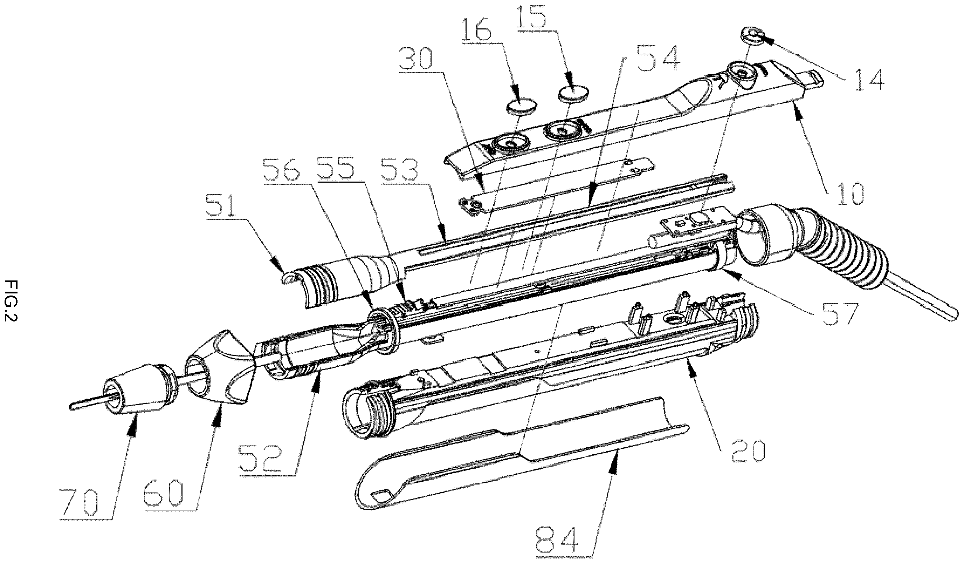

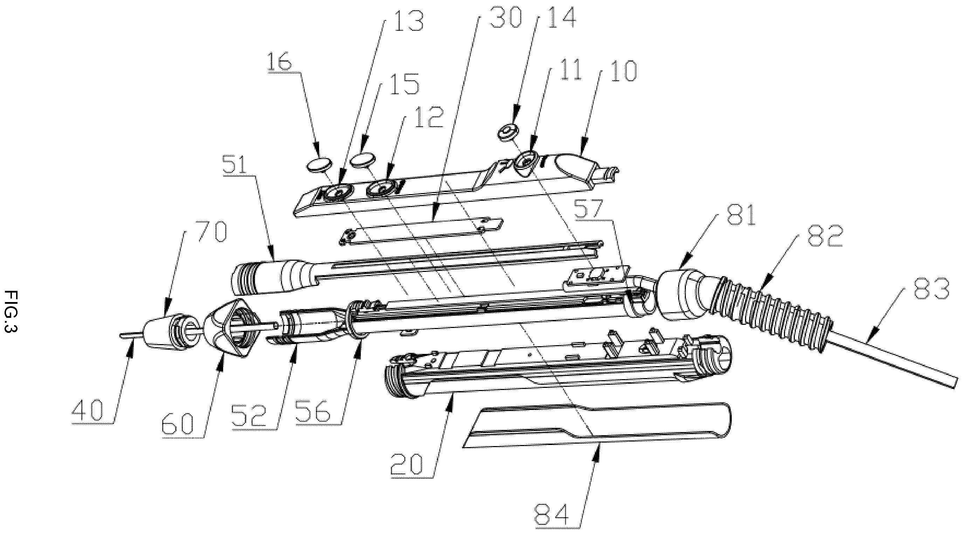

As shown in Figures 1-3, Figure 1 shows a three-dimensional structure of the smoke-absorbing high-frequency surgical knife with illumination function and capable of locking the telescopic length, and Figures 2-3 respectively show explosive structures of the smoke-absorbing high-frequency surgical knife with illumination function and capable of locking the telescopic length from different angles. Preferably, the smoke-absorbing high-frequency surgical knife with illumination function and capable of locking the telescopic length comprises a surgical knife upper shell 10 and a surgical knife main body 20, wherein the surgical knife upper shell 10 is located above the surgical knife main body 20, and is embedded with the surgical knife main body 20. A first accommodating chamber is arranged between the surgical knife upper shell 10 and the surgical knife main body 20, and two double-sided PCBs are arranged in the first accommodating chamber, wherein the circuit on one side of one double-sided PCB 30 is used for switching on/off the high-frequency electrode 40 (the output of the electrode) according to the second or third control signals triggered by the user, the circuit on the other side of the aforesaid double-sided PCB is used for turning on/off an LED light source, and the other double-sided PCB is used for turning on/off the SMD LED lamp beads (the output of the LED lamp beads) according to a first control signal triggered by the user. A second accommodating chamber is arranged in the surgical knife main body 20. The smoke-absorbing high-frequency surgical knife with illumination function and capable of locking the telescopic length further comprises a smoke-absorbing tube 50, a locking mechanism 60, a light-emitting element 70, and a high-frequency electrode 40, wherein a portion of the smoke-absorbing tube 50 is located in the second accommodating chamber, the portion (located in the second accommodating chamber) of the smoke-absorbing tube 50 is provided with a slide groove for the smoke-absorbing tube 53, and the aforesaid SMD LED lamp beads are arranged in an annular shape. The other portion of the smoke-absorbing tube 50 is located outside the surgical knife main body 20, and the portion (located outside the surgical knife main body 20) of the smoke-absorbing tube 50 is internally connected with the locking mechanism 60. The light-emitting element 70 is internally connected with the smoke-absorbing tube 50, and the inner surface of the smoke-absorbing tube 50 integrally extends to form an electrode fixing seat (not shown in the figure). The high-frequency electrode 40 is coaxially arranged with the light-emitting element 70. One end of the high-frequency electrode 40 is located on the outer side of the light-emitting element 70, and keeps the absolute distance from the light-emitting element 70 unchanged. The other end of the high-frequency electrode 40 passes through the light-emitting element 70 and is internally connected to the aforesaid electrode fixing seat. Thus, when the smoke-absorbing tube stretches and retracts relative to the axial direction of the surgical knife main body 20, the high-frequency electrode 40, the light-emitting element 70 and the SMD LED lamp beads synchronously stretch and retract relative to the axial direction of the surgical knife main body 20.

Further, the smoke-absorbing high-frequency surgical knife with illumination function and capable of locking the telescopic length comprises a high-frequency conductive copper groove 54. The high-frequency conductive copper groove 54 is located between the surgical knife main body 20 and the smoke-absorbing tube 50, and the high-frequency conductive copper groove 54 is arranged in the axial direction of the surgical knife main body 20. The high-frequency conductive copper groove 54 interacts with the slide groove for smoke-absorbing tube 53 of the smoke-absorbing tube 50, thereby enabling the smoke-absorbing tube 50 to slide along the high-frequency conductive copper groove 54 with the power supplied by the high-frequency conductive copper groove 54 for the whole process. In other words, the smoke-absorbing tube 50 synchronously stretches and retracts along the axial direction of the surgical knife main body 20, thereby propelling the high-frequency electrode 40 and the double-sided PCB 30 with a plurality of SMD LED lamp beads to synchronously stretch and retract relative to the axial direction of the surgical knife main body 20.

Further, the smoke-absorbing high-frequency surgical knife with illumination function and capable of locking the telescopic length comprises a buckle 55 and an annular sealing ring 56. The locking mechanism 60 comprises an outer locking mechanism, and the annular sealing ring 56 is internally arranged in the outer locking mechanism.

The buckle 55 is provided with a buckle lower surface protruding portion. One end of the buckle 55 is internally connected with the outer locking mechanism, and the other end of the buckle 55 is embedded into the surgical knife main body 20. The buckle lower surface protruding portion of the buckle 55 is embedded into the slide groove for the smoke-absorbing tube 53.

The middle of the upper surface of the buckle 55 is provided with a buckle upper surface protruding portion. A plurality of grooves with different depths are formed in one side of the buckle upper surface protruding portion arranged on the upper surface of the buckle 55, and a reverse slope is provided on the other side of the buckle upper surface protruding portion arranged on the upper surface of the buckle 55. The tail end of the buckle 55 that is provided with the plurality of grooves with different depths abuts against the annular sealing ring 56.

The inner surface of the outer locking mechanism is provided with a plurality of convex rings with different heights, and each of the grooves with different depths on the upper surface of the buckle 55 respectively interacts with each of the convex rings with different heights on the inner surface of the outer locking mechanism, thereby finely adjusting the locking degree of the outer locking mechanism so that the locking function can be achieved at any position.

One end of the surgical knife upper shell 10 is provided with a contracting portion that gradually contracts towards the axis, and the reverse slope of the upper surface of the buckle 55 is attached to the contracting portion of the surgical knife upper shell 10. The outer locking mechanism is provided with an outer locking mechanism outer main body, and an outer locking mechanism inner protruding portion is integrally formed with the outer locking mechanism outer main body. The outer locking mechanism inner protruding portion is provided with a through-hole, and the smoke-absorbing tube 50 is internally connected with the through-hole of the outer locking mechanism inner protruding portion.

An outer locking mechanism accommodating chamber is formed between the outer locking mechanism outer main body and the outer locking mechanism inner protruding portion, and the end portion of one end of the surgical knife main body 20 is internally connected to the outer locking mechanism accommodating chamber.

One side of the outer locking mechanism is in threaded connection with the light-emitting element 70, and the outer locking mechanism outer main body of the outer locking mechanism is in threaded connection with one end of the surgical knife main body 20.

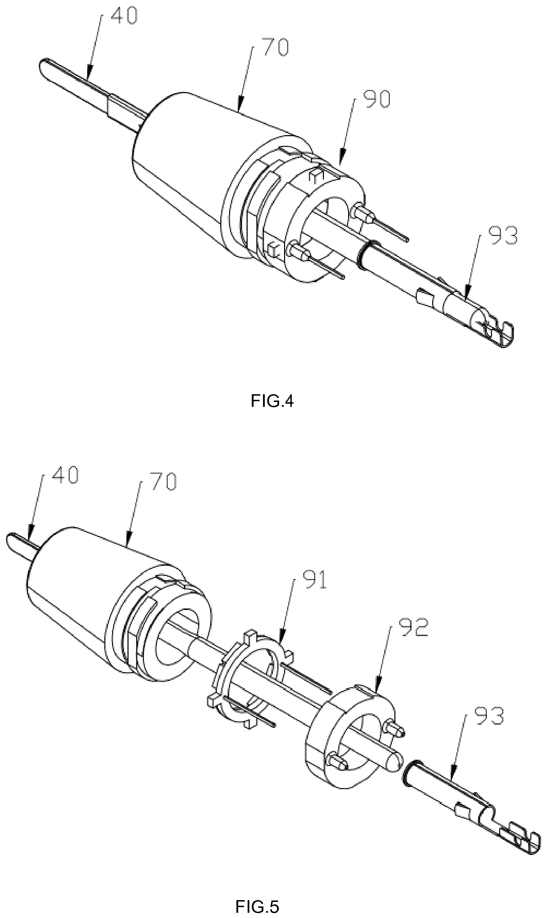

It should be noted that, on the basis of the preferred embodiment, the present invention further discloses a first modification of the preferred embodiment, wherein the aforesaid first modification is basically the same as the preferred embodiment, and the difference between is the illuminating component 90 (except for the SMD LED lamp beads) of the preferred embodiment is further modified as follows:
As shown in Figures 4-5, the illuminating component 90 and the light-emitting element 70 are coaxially arranged. The illuminating component 90 comprises an illuminating ring 91 and an illuminating body 92, and the illuminating ring 91 is internally connected with the illuminating body 92.

The smoke-absorbing high-frequency surgical knife with illumination function and capable of locking the telescopic length further comprises an SOC part 93, and the SOC part 93 is connected with the end portion of one end of the high-frequency electrode 40 in a sleeved mode.

The smoke-absorbing tube 50 comprises a smoke-absorbing tube upper portion 51 and a smoke-absorbing tube lower portion 52 that partially covers the upper portion 51 of the smoke-absorbing tube. The upper portion 51 of the smoke-absorbing tube is connected with the lower portion 52 of the smoke-absorbing tube through ultrasonic welding.

The smoke-absorbing high-frequency surgical knife with illumination function and capable of locking the telescopic length further comprises a bent joint 81, a hose 82 and a current carrying wire 83, wherein one side of the bent joint 81 is simultaneously connected to the surgical knife upper shell 10 and the surgical knife main body 20 in a sleeved mode, and the other side of the bent joint 81 is internally connected with the hose 82. The current carrying wire 83 extends into the first accommodating chamber formed between the surgical knife upper shell 10 and the surgical knife main body 20 through the bent joint 81.

The smoke-absorbing high-frequency surgical knife with illumination function and capable of locking the telescopic length further comprises an anti-slip sleeve 84, wherein the surgical knife main body 20 is covered by the anti-slip sleeve 84, thereby improving the overall hand feeling when holding the smoke-absorbing high-frequency surgical knife with illumination function and capable of locking the telescopic length, and increasing the anti-slip performance of the smoke-absorbing high-frequency surgical knife with illumination function and capable of locking the telescopic length.

The smoke-absorbing high-frequency surgical knife with illumination function and capable of locking the telescopic length further comprises an illuminating button 14, an electric coagulation button 15 and an electric cutting button 16, wherein the illuminating button 14 is used for inputting the first control signal, the electric coagulation button 15 is used for inputting the second control signal, and the electric cutting button 16 is used for inputting the third control signal.

Further, the surgical knife upper shell 10 is provided with a first positioning hole 11, a second positioning hole 12 and a third positioning hole 13, wherein the illuminating button 14, the electric coagulation button 15 and the electric cutting button 16 are respectively embedded into the first positioning hole 11, the second positioning hole 12 and the third positioning hole 13.

According to the aforesaid preferred embodiment, the smoke-absorbing high-frequency surgical knife with illumination function and capable of locking the telescopic length of the present invention has the following technical features:
Frist, the smoke-absorbing high-frequency surgical knife with illumination function and capable of locking the telescopic length of the present invention adopts an internal illumination mode. When the smoke-absorbing tube stretches and retracts, the high-frequency electrode, the light-emitting element, and the SMD LED lamp beads synchronously stretch and retract along with the electrode;
Second, the illuminating component (SMD LED lamp beads) is close to the position where the high-frequency electrode effectively acts, and keeps the absolute distance unchanged, achieving a stable illumination intensity at the acting position of the high-frequency electrode;
Third, the illuminating component (SMD LED lamp beads) annularly and uniformly emits light, ensuring the surgical spot is shadow-free;
Fourth, in addition to the aforesaid functions, the electric knife of the present invention is also capable of discharging the smoke generated during surgery;
Fifth, the light-emitting element adopts an internal illumination mode, avoiding the size of the electric knife from being increased while synchronously stretching and retracting along the smoke-absorbing tube;
Sixth, the SMD LED lamp beads are annularly arranged on the double-sided PCB, and the light rays are emitted out through the light-emitting element, thus achieving a uniform annular illumination so that the shadow can be prevented from appearing;
Seventh, the distance between the light-emitting element and the head portion of the electrode is smaller than 30 mm, and the distance is kept absolutely unchanged, achieving a high illumination intensity;
Eighth, the SMD LED lamp beads are wrapped by a light-transmitting plastic material, achieving effects such as insulation and water resistance;
Ninth, the power supply line of the SMD LED lamp beads and the high-frequency line of the high-frequency electrode are mutually independent and insulated from each other so that the influence between the lines can be avoided;
Tenth, the smoke-absorbing tube is telescopic, and can be locked at any position;
Eleventh, the locking mechanism is provided with an outer locking mode and an inner locking mode.

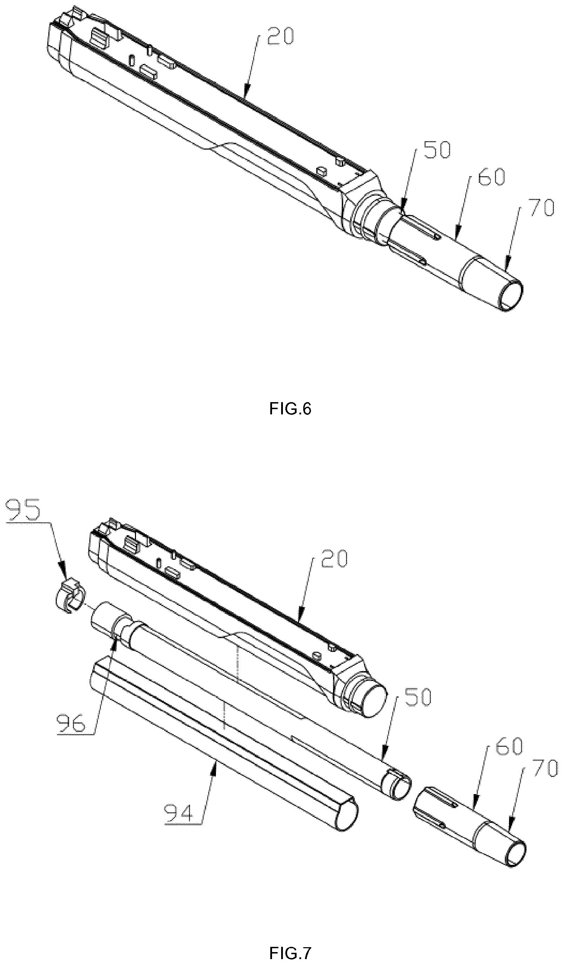

It should be noted that, on the basis of the disclosed preferred embodiment, the present invention further discloses a second modification of the preferred embodiment, wherein the aforesaid second modification is basically the same as the preferred embodiment, and the difference between them is that the outer locking mechanism of the preferred embodiment is replaced by an inner locking mechanism, which is briefly described hereinafter:
As shown in Figures 6-7, the locking mechanism 60 further comprises an inner locking mechanism.

Specifically, the smoke-absorbing tube 50 is internally connected with a conductive special-shaped tube 94, and one end of the smoke-absorbing tube 50 is sleeved with an eccentric tensioning ring 95. The end portion of the smoke-absorbing tube 50 sleeved with the eccentric tensioning ring 95 is provided with an eccentric groove 96 annularly arranged along the outer surface of the end portion of the smoke-absorbing tube 50. The eccentric tensioning ring 95 is partially and externally connected with the eccentric groove 96 of the smoke-absorbing tube 50. The distance between the outer periphery of the eccentric groove 96 and the inner surface of the conductive special-shaped tube 94 varies periodically in the radial direction of the smoke-absorbing tube 50. In other words, the gap space between the outer periphery of the eccentric groove 96 and the inner surface of the conductive special-shaped tube 94 is not consistent, and the space on one side of the eccentric groove 96 is (slightly) greater than that on the other side of the eccentric groove 96. When the inner locking function is needed, the user can rotate the smoke-absorbing tube 50 in the radial direction, thus allowing the gap between the eccentric tensioning ring 95 and the conductive special-shaped tube 94 to vary periodically. When the rotation amplitude of the smoke-absorbing tube 50 in the radial direction is large, the eccentric tensioning ring 95 is completely attached to the inner surface of the conductive special-shaped tube 94 to achieve the purpose of inner locking.

Twelfth, the telescopic distance of the smoke-absorbing tube is greater than 9.5cm;

Thirteenth, the outer surface of the smoke-absorbing tube is provided with scales for accurately indicating the telescopic distance.

According to aforesaid preferred embodiment, the smoke-absorbing high-frequency surgical knife with illumination function and capable of locking the telescopic length disclosed in the present invention further comprises a first modified implementation way of the aforesaid preferred embodiment.

Specifically, the aforesaid first modified implementation way is basically the same as the preferred embodiment, and the difference between them lies in the follows:
First, the specific implementation way of achieving the smoke-absorbing function is different. Specifically, the light-emitting element 70, the smoke-absorbing tube 50, the surgical knife main body 20, the bent joint 81 and the hose 82 of the smoke-absorbing high-frequency surgical knife with illumination function and capable of locking the telescopic length form a smoke-absorbing passage in sequence.

## Claims

1. A smoke-absorbing high-frequency surgical knife with illumination function and capable of locking a telescopic length, comprising:
a surgical knife upper shell (10), and
a surgical knife main body (20), wherein the surgical knife upper shell is located above the surgical knife main body, and is embedded with the surgical knife main body,
wherein a first accommodating chamber is arranged between the surgical knife upper shell and the surgical knife main body, and two double-sided PCBs (30) are arranged in the first accommodating chamber, wherein a circuit on one side of one double-sided PCB is adapted to be used for switching on/off a high-frequency current of a high-frequency electrode (40) according to second or third control signals triggered by the user, the circuit on the other side of the aforesaid double-sided PCB is adapted to be used for turning on/off an LED light source, and the other double-sided PCB is used for turning on/off SMD LED lamp beads according to a first control signal triggered by the user, wherein a second accommodating chamber is arranged in the surgical knife main body, wherein the smoke-absorbing high-frequency surgical knife with illumination function and capable of locking the telescopic length further comprises a smoke-absorbing tube (50), a locking mechanism (60), a light-emitting element (70), and a high-frequency electrode, wherein a portion of the smoke-absorbing tube is located in the second accommodating chamber, the portion of the smoke-absorbing tube is provided with a slide groove for the smoke-absorbing tube (53), and the SMD LED lamp beads are arranged in an annular shape in an illuminating component (90), wherein the other portion of the smoke-absorbing tube is located outside the surgical knife main body, and the portion of the smoke-absorbing tube is internally connected with the locking mechanism, wherein the light-emitting element is internally connected with the smoke-absorbing tube, and an inner surface of the smoke-absorbing tube integrally extends to form an electrode fixing seat, wherein the high-frequency electrode is coaxially arranged with the light-emitting element, wherein one end of the high-frequency electrode is located on the outer side of the light-emitting element, and is adapted to keep the absolute distance from the light-emitting element unchanged, wherein the other end of the high-frequency electrode passes through the light-emitting element and is internally connected to the electrode fixing seat, thereby enabling the high-frequency electrode, the light-emitting element and the SMD LED lamp beads to synchronously stretch and retract relative to the axial direction of the surgical knife main body when the smoke-absorbing tube stretches and retracts relative to the axial direction of the surgical knife main body; wherein a power supply line of the SMD LED lamp beads and a high-frequency line of the high-frequency electrode are mutually independent, further enhancing the stability and safety of the smoke-absorbing high-frequency surgical knife with illumination function and capable of locking the telescopic length.

2. The smoke-absorbing high-frequency surgical knife with illumination function and capable of locking the telescopic length of claim 1, wherein the SMD LED lamp beads are wrapped by a light-transmitting plastic material, thereby achieving a water-resistant effect.

3. The smoke-absorbing high-frequency surgical knife with illumination function and capable of locking the telescopic length of claim 1 or 2, wherein the smoke-absorbing high-frequency surgical knife with illumination function and capable of locking the telescopic length further comprises a buckle (55) and an annular sealing ring (56), wherein the locking mechanism comprises an outer locking mechanism, and the annular sealing ring is internally arranged in the outer locking mechanism, wherein the buckle is provided with a buckle lower surface protruding portion, wherein one end of the buckle is internally connected with the outer locking mechanism, and the other end of the buckle is embedded into the surgical knife main body, wherein the buckle lower surface protruding portion of the buckle is embedded into the slide groove for the smoke-absorbing tube.

4. The smoke-absorbing high-frequency surgical knife with illumination function and capable of locking the telescopic length of claim 1 or 2, wherein the locking mechanism comprises an inner locking mechanism, and the inner locking mechanism comprises a conductive tube (94), wherein the smoke-absorbing tube is internally connected with a conductive tube, and one end of the smoke-absorbing tube is sleeved with an eccentric tensioning ring (95), wherein the end portion of the smoke-absorbing tube sleeved with the eccentric tensioning ring is provided with an eccentric groove (96) annularly arranged along the outer surface of the end portion of the smoke-absorbing tube, wherein the eccentric tensioning ring is partially and externally connected with the eccentric groove of the smoke-absorbing tube, wherein the distance between the outer periphery of the eccentric groove and the inner surface of the conductive special-shaped tube varies periodically in the radial direction of the smoke-absorbing tube.

5. The smoke-absorbing high-frequency surgical knife with illumination function and capable of locking the telescopic length of claim 3, wherein the middle of the upper surface of the buckle is provided with a buckle upper surface protruding portion, wherein a plurality of grooves with different depths are formed in one side of the buckle upper surface protruding portion arranged on the upper surface of the buckle, and a reverse slope is provided on the other side of the buckle upper surface protruding portion arranged on the upper surface of the buckle, wherein the tail end of the buckle that is provided with the plurality of grooves with different depths abuts against the annular sealing ring, wherein the inner surface of the outer locking mechanism is provided with a plurality of convex rings with different heights, and each of the grooves with different depths on the upper surface of the buckle respectively interacts with each of the convex rings with different heights on the inner surface of the outer locking mechanism.

6. The smoke-absorbing high-frequency surgical knife with illumination function and capable of locking the telescopic length of claim 1, wherein the smoke-absorbing high-frequency surgical knife with illumination function and capable of locking the telescopic length further comprises a fixing seat of the conductive spring of the smoke-absorbing tube and a high-frequency conductive spring, wherein one end of the smoke-absorbing tube is internally connected with the fixing seat of the conductive spring of the smoke-absorbing tube , wherein one end of the high-frequency conductive spring is internally connected with the fixing seat of the conductive spring of the smoke-absorbing tube , and the other end of the high-frequency conductive spring is internally connected with a high-frequency conductive copper groove (54).

7. The smoke-absorbing high-frequency surgical knife with illumination function and capable of locking the telescopic length of claim 1, wherein the smoke-absorbing high-frequency surgical knife with illumination function and capable of locking the telescopic length further comprises an anti-slip sleeve (84), and the surgical knife main body is covered by the anti-slip sleeve.

8. The smoke-absorbing high-frequency surgical knife with illumination function and capable of locking the telescopic length of claim 1, wherein the smoke-absorbing high-frequency surgical knife with illumination function and capable of locking the telescopic length further comprises a bent joint (81), a hose (82) and a current carrying wire (83), wherein one side of the bent joint is simultaneously connected to the surgical knife upper shell and the surgical knife main body in a sleeved mode, and the other side of the bent joint is internally connected with the hose, wherein the current carrying wire extends into the first accommodating chamber formed between the surgical knife upper shell and the surgical knife main body through the bent joint.

9. The smoke-absorbing high-frequency surgical knife with illumination function and capable of locking the telescopic length of claim 1, wherein the smoke-absorbing high-frequency surgical knife with illumination function and capable of locking the telescopic length further comprises an illuminating button (14), an electric coagulation button and an electric cutting button, wherein the illuminating button is used for inputting the first control signal, the electric coagulation button is used for inputting the second control signal, and the electric cutting button is adapted to be used for inputting the third control signal, wherein the surgical knife upper shell is provided with a first positioning hole, a second positioning hole and a third positioning hole, wherein the illuminating button, the electric coagulation button and the electric cutting button are respectively embedded into the first positioning hole, the second positioning hole and the third positioning hole.

## Patentansprüche

1. Rauchabsorbierendes chirurgisches Hochfrequenzmesser mit Beleuchtungsfunktion, das eine ausziehbare Länge arretieren kann, umfassend:
eine obere Hülse (10) des chirurgischen Messers und
einen Hauptkörper (20) des chirurgischen Messers, wobei die obere Hülse des chirurgischen Messers sich über dem Hauptkörper des chirurgischen Messers befindet und mit dem Hauptkörper des chirurgischen Messers eingebettet ist,
wobei eine erste Aufnahmekammer zwischen der oberen Hülse des chirurgischen Messers und dem Hauptkörper des chirurgischen Messers angeordnet ist und zwei doppelseitige PCB (30) in der ersten Aufnahmekammer angeordnet sind, wobei eine Schaltung auf einer Seite einer doppelseitigen PCB dazu eingerichtet ist, zum Ein-/Ausschalten eines Hochfrequenzstroms einer Hochfrequenzelektrode (40) gemäß einem zweiten oder einem dritten Steuersignal, das von dem Benutzer ausgelöst wird,
verwendet zu werden, die Schaltung auf der anderen Seite der oben erwähnten doppelseitigen PCB dazu eingerichtet ist, zum Ein-/Ausschalten einer LED-Lichtquelle verwendet zu werden, und die andere doppelseitige PCB zum Ein-/Ausschalten von SMD-LED-Lampenperlen gemäß einem ersten Steuersignal, das von dem Benutzer ausgelöst wird,
verwendet wird, wobei eine zweite Aufnahmekammer in dem Hauptkörper des chirurgischen Messers angeordnet ist,
wobei das rauchabsorbierende chirurgische Hochfrequenzmesser mit Beleuchtungsfunktion, das die ausziehbare Länge arretieren kann, weiterhin eine rauchabsorbierende Röhre (50), einen Arretiermechanismus (60), ein lichtemittierendes Element (70) und eine Hochfrequenzelektrode umfasst, wobei ein Abschnitt der rauchabsorbierenden Röhre sich in der zweiten Aufnahmekammer befindet, wobei der Abschnitt der rauchabsorbierenden Röhre mit einer Gleitnut für die rauchabsorbierende Röhre (53) versehen ist und die SMD-LED-Lampenperlen in einer Ringform in einer Beleuchtungskomponente (90) angeordnet sind, wobei der andere Abschnitt der rauchabsorbierenden Röhre sich außerhalb des Hauptkörpers des chirurgischen Messers befindet und der Abschnitt der rauchabsorbierenden Röhre intern mit dem Arretiermechanismus verbunden ist, wobei das lichtemittierende Element intern mit der rauchabsorbierenden Röhre verbunden ist und eine Innenfläche der rauchabsorbierenden Röhre sich integral erstreckt, um einen Elektrodenfixierungssitz zu bilden,
wobei die Hochfrequenzelektrode koaxial mit dem lichtemittierenden Element angeordnet ist, wobei ein Ende der Hochfrequenzelektrode sich auf der Außenseite des lichtemittierenden Elements befindet und dazu eingerichtet ist, den absoluten Abstand von dem lichtemittierenden Element unverändert zu halten, wobei das andere Ende der Hochfrequenzelektrode durch das lichtemittierende Element verläuft und intern mit dem Elektrodenfixierungssitz verbunden ist, wodurch ermöglicht wird, dass die Hochfrequenzelektrode, das lichtemittierende Element und
die SMD-LED-Lampenperlen sich in Bezug auf die Achsenrichtung des Hauptkörpers des chirurgischen Messers synchron auszustrecken und zurückziehen, wenn die rauchabsorbierende Röhre sich in Bezug auf die Achsenrichtung des Hauptkörpers des chirurgischen Messers ausstreckt und zurückzieht; wobei eine Stromleitung der SMD-LED-Lampenperlen und eine Hochfrequenzleitung der Hochfrequenzelektrode voneinander unabhängig sind, wodurch die Stabilität und die Sicherheit des rauchabsorbierenden chirurgischen Hochfrequenzmessers mit Beleuchtungsfunktion, das die ausziehbare Länge arretieren kann, weiter verbessert wird.

2. Rauchabsorbierendes chirurgisches Hochfrequenzmesser mit Beleuchtungsfunktion, das die ausziehbare Länge arretieren kann, nach Anspruch 1, wobei die SMD-LED-Lampenperlen durch ein lichtübertragendes Kunststoffmaterial eingehüllt sind, wodurch ein wasserbeständiger Effekt erzielt wird.

3. Rauchabsorbierendes chirurgisches Hochfrequenzmesser mit Beleuchtungsfunktion, das die ausziehbare Länge arretieren kann, nach Anspruch 1 oder 2, wobei das rauchabsorbierende chirurgische Hochfrequenzmesser mit Beleuchtungsfunktion, das die ausziehbare Länge arretieren kann, weiterhin eine Schnalle (55) und einen ringförmigen Dichtring (56) umfasst, wobei der Arretiermechanismus einen äußeren Arretiermechanismus umfasst und der ringförmige Dichtring intern in dem äußeren Arretiermechanismus angeordnet ist, wobei die Schnalle mit einem vorstehenden Abschnitt einer unteren Schnallenfläche versehen ist, wobei ein Ende der Schnalle intern mit dem äußeren Arretiermechanismus verbunden ist und das andere Ende der Schnalle in den Hauptkörper des chirurgischen Messers eingebettet ist, wobei der vorstehende Abschnitt der unteren Schnallenfläche der Schnalle in die Gleitnut für die rauchabsorbierende Röhre eingebettet ist.

4. Rauchabsorbierendes chirurgisches Hochfrequenzmesser mit Beleuchtungsfunktion, das die ausziehbare Länge arretieren kann, nach Anspruch 1 oder 2, wobei der Arretiermechanismus einen inneren Arretiermechanismus umfasst und der innere Arretiermechanismus eine leitfähige Röhre (94) umfasst, wobei die rauchabsorbierende Röhre intern mit der leitfähigen Röhre verbunden ist und ein Ende der rauchabsorbierenden Röhre mit einem exzentrischen Klemmring (95) umhüllt ist, wobei der Endabschnitt der rauchabsorbierenden Röhre, der mit dem exzentrischen Klemmring umhüllt ist, mit einer exzentrischen Nut (96) versehen ist, die ringförmig entlang der Außenfläche des Endabschnitts der rauchabsorbierenden Röhre angeordnet ist, wobei der exzentrische Klemmring zum Teil und extern mit der exzentrischen Nut der rauchabsorbierenden Röhre verbunden ist, wobei der Abstand zwischen dem Außenumfang der exzentrischen Nut und der Innenfläche der leitfähigen, speziell geformten Röhre periodisch in der Radialrichtung der rauchabsorbierenden Röhre variiert.

5. Rauchabsorbierendes chirurgisches Hochfrequenzmesser mit Beleuchtungsfunktion, das die ausziehbare Länge arretieren kann, nach Anspruch 3, wobei die Mitte der oberen Fläche der Schnalle mit einem vorstehenden Abschnitt der oberen Schnallenfläche versehen ist, wobei mehrere Nuten mit unterschiedlichen Tiefen in einer Seite des vorstehenden Abschnitts der oberen Schnallenfläche auf der oberen Fläche der Schnalle angeordnet sind und eine umgekehrte Schräge auf der anderen Seite des vorstehenden Abschnitts der oberen Schnallenfläche, der auf der oberen Fläche der Schnalle angeordnet ist, vorgesehen ist, wobei das hintere Ende der Schnalle, das mit den mehreren Nuten mit unterschiedlichen Tiefen versehen ist, gegen den ringförmigen Dichtring anstößt, wobei die Innenfläche des äußeren Arretiermechanismus mit mehreren konvexen Ringen mit unterschiedlichen Höhen versehen ist und jede der Nuten mit unterschiedlichen Tiefen auf der oberen Fläche der Schnalle jeweils mit jedem der konvexen Ringe mit unterschiedlichen Höhen auf der Innenfläche des äußeren Arretiermechanismus interagiert.

6. Rauchabsorbierendes chirurgisches Hochfrequenzmesser mit Beleuchtungsfunktion, das die ausziehbare Länge arretieren kann, nach Anspruch 1, wobei das rauchabsorbierende chirurgische Hochfrequenzmesser mit Beleuchtungsfunktion, das die ausziehbare Länge arretieren kann, weiterhin einen Fixierungssitz der leitfähigen Feder der rauchabsorbierenden Röhre und eine leitfähige Hochfrequenzfeder umfasst, wobei ein Ende der rauchabsorbierenden Röhre intern mit dem Fixierungssitz der leitfähigen Feder der rauchabsorbierenden Röhre verbunden ist, wobei ein Ende der leitfähigen Hochfrequenzfeder intern mit dem Fixierungssitz der leitfähigen Feder der rauchabsorbierenden Röhre verbunden ist und das andere Ende der leitfähigen Hochfrequenzfeder intern mit einer leitfähigen Hochfrequenzkupfernut (54) verbunden ist.

7. Rauchabsorbierendes chirurgisches Hochfrequenzmesser mit Beleuchtungsfunktion, das die ausziehbare Länge arretieren kann, nach Anspruch 1, wobei das rauchabsorbierende chirurgische Hochfrequenzmesser mit Beleuchtungsfunktion, das die ausziehbare Länge arretieren kann, weiterhin eine Antirutschhülle (84) umfasst und der Hauptkörper des chirurgischen Messers durch die Antirutschhülle bedeckt ist.

8. Rauchabsorbierendes chirurgisches Hochfrequenzmesser mit Beleuchtungsfunktion, das die ausziehbare Länge arretieren kann, nach Anspruch 1, wobei das rauchabsorbierende chirurgische Hochfrequenzmesser mit Beleuchtungsfunktion, das die ausziehbare Länge arretieren kann, weiterhin ein abgewinkeltes Gelenk (81), einen Schlauch (82) und einen stromführenden Draht (83) umfasst, wobei eine Seite des abgewinkelten Gelenks gleichzeitig mit der oberen Hülse des chirurgischen Messers und dem Hauptkörper des chirurgischen Messers in einem umhüllten Modus verbunden ist und das andere Ende des abgewinkelten Gelenks intern mit dem Schlauch verbunden ist, wobei der stromführende Draht sich in die erste Aufnahmekammer, die zwischen der oberen Hülse des chirurgischen Messers und dem Hauptkörper des chirurgischen Messers gebildet ist, durch das abgewinkelte Gelenk erstreckt.

9. Rauchabsorbierendes chirurgisches Hochfrequenzmesser mit Beleuchtungsfunktion, das die ausziehbare Länge arretieren kann, nach Anspruch 1, wobei das rauchabsorbierende chirurgische Hochfrequenzmesser mit Beleuchtungsfunktion, das die ausziehbare Länge arretieren kann, weiterhin einen Beleuchtungsknopf (14), einen elektrischen Koagulationsknopf und einen elektrischen Schneidknopf umfasst, wobei der Beleuchtungsknopf zum Eingeben des ersten Steuersignals verwendet wird, der elektrische Koagulationsknopf zum Eingeben des zweiten Steuersignals verwendet wird und der elektrische Schneidknopf dazu eingerichtet ist, zum Eingeben des dritten Steuersignals verwendet zu werden, wobei die obere Hülse des chirurgischen Messers mit einem ersten Positionierungsloch, einem zweiten Positionierungsloch und einem dritten Positionierungsloch versehen ist, wobei der Beleuchtungsknopf, der elektrische Koagulationsknopf und der elektrische Schneidknopf jeweils in das erste Positionierungsloch, das zweite Positionierungsloch und das dritte Positionierungsloch eingebettet sind.

## Revendications

1. Bistouri à haute fréquence absorbant la fumée à fonction d'éclairage et apte à verrouiller une longueur télescopique, comprenant :
une coque supérieure de bistouri (10), et
un corps principal de bistouri (20), dans lequel la coque supérieure de bistouri est située au-dessus du corps principal de bistouri, et est intégrée au corps principal de bistouri, dans lequel une première chambre de réception est agencée entre la coque supérieure de bistouri et le corps principal de bistouri, et deux cartes de circuits imprimés à double face (30) sont agencées dans la première chambre de réception, dans lequel un circuit sur une face d'une carte de circuits imprimés à double face est adapté à être utilisé pour mettre en marche/couper un courant à haute fréquence d'une électrode à haute fréquence (40) en fonction de deuxième ou troisième signaux de commande déclenchés par l'utilisateur, le circuit sur l'autre face de ladite carte de circuits imprimés à double face est adapté à être utilisé pour allumer/éteindre une source de lumière à DEL, et l'autre carte de circuits imprimés à double face est utilisée pour allumer/éteindre des manchons de verre de lampe à DEL CMS en fonction d'un premier signal de commande déclenché par l'utilisateur,
dans lequel une deuxième chambre de réception est agencée dans le corps principal de bistouri, dans lequel le bistouri à haute fréquence absorbant la fumée à fonction d'éclairage et apte à verrouiller la longueur télescopique comprend en outre un tube d'absorption de fumée (50), un mécanisme de verrouillage (60), un élément électroluminescent (70), et une électrode à haute fréquence, dans lequel une partie du tube absorbant la fumée est située dans la deuxième chambre de réception, la partie du tube absorbant la fumée est pourvue d'une rainure de glissement pour le tube absorbant la fumée (53), et les manchons de verre de lampe à DEL CMS sont agencés selon une forme annulaire dans un composant d'éclairage (90),
dans lequel l'autre partie du tube absorbant la fumée est située à l'extérieur du corps principal de bistouri, et la partie du tube absorbant la fumée est reliée de manière interne au mécanisme de verrouillage, dans lequel l'élément électroluminescent est relié de manière interne au tube absorbant la fumée, et une surface intérieure du tube absorbant la fumée s'étend d'une seule pièce pour former un siège de fixation d'électrode, dans lequel l'électrode à haute fréquence est agencée de manière coaxiale avec l'élément électroluminescent, dans lequel une extrémité de l'électrode à haute fréquence est située sur la face extérieure de l'élément électroluminescent, et
est adaptée à garder la distance absolue à partir de l'élément électroluminescent inchangée, dans lequel l'autre extrémité de l'électrode à haute fréquence passe à travers l'élément électroluminescent et est reliée de manière interne au siège de fixation d'électrode, activant alors l'électrode à haute fréquence, l'élément électroluminescent et les manchons de verre de lampe à DEL CMS pour s'étirer et se rétracter de manière synchrone par rapport à la direction axiale du corps principal de bistouri lorsque le tube absorbant la fumée s'étire et se rétracte par rapport à la direction axiale du corps principal de bistouri ; dans lequel une ligne d'alimentation électrique des manchons de verre de lampe à DEL CMS et une ligne à haute fréquence de l'électrode à haute fréquence sont mutuellement indépendantes,
améliorant en outre la stabilité et la sécurité du bistouri à haute fréquence absorbant la fumée à fonction d'éclairage et apte à verrouiller la longueur télescopique.

2. Bistouri à haute fréquence absorbant la fumée à fonction d'éclairage et apte à verrouiller la longueur télescopique selon la revendication 1, dans lequel les manchons de verre de lampe à DEL CMS sont enveloppés d'une matière plastique transmettant la lumière, obtenant ainsi un effet résistant à l'eau.

3. Bistouri à haute fréquence absorbant la fumée à fonction d'éclairage et apte à verrouiller la longueur télescopique selon la revendication 1 ou 2, dans lequel le bistouri à haute fréquence absorbant la fumée à fonction d'éclairage et apte à verrouiller la longueur télescopique comprend en outre une boucle (55) et une bague d'étanchéité annulaire (56), dans lequel le mécanisme de verrouillage comprend un mécanisme de verrouillage extérieur, et la bague d'étanchéité annulaire est agencée de manière interne dans le mécanisme de verrouillage extérieur, dans lequel la boucle est pourvue d'une partie saillante de surface inférieure de boucle, dans lequel une extrémité de la boucle est reliée de manière interne au mécanisme de verrouillage extérieur, et l'autre extrémité de la boucle est intégrée dans le corps principal de bistouri, dans lequel la partie saillante de surface inférieure de boucle de la boucle est intégrée dans la rainure de glissement pour le tube absorbant la fumée.

4. Bistouri à haute fréquence absorbant la fumée à fonction d'éclairage et apte à verrouiller la longueur télescopique selon la revendication 1 ou 2, dans lequel le mécanisme de verrouillage comprend un mécanisme de verrouillage intérieur, et le mécanisme de verrouillage intérieur comprend un tube conducteur (94), dans lequel le tube absorbant la fumée est relié de manière interne à un tube conducteur, et une extrémité du tube absorbant la fumée est manchonnée avec une bague de tension excentrique (95), dans lequel la partie d'extrémité du tube absorbant la fumée manchonnée avec la bague de tension excentrique est pourvue d'une rainure excentrique (96) agencée de manière annulaire le long de la surface extérieure de la partie d'extrémité du tube absorbant la fumée, dans lequel la bague de tension excentrique est reliée partiellement et de manière externe à la rainure excentrique du tube absorbant la fumée, dans lequel la distance entre la périphérie extérieure de la rainure excentrique et la surface intérieure du tube conducteur de forme spéciale varie de manière périodique dans la direction radiale du tube absorbant la fumée.

5. Bistouri à haute fréquence absorbant la fumée à fonction d'éclairage et apte à verrouiller la longueur télescopique selon la revendication 3, dans lequel le centre de la surface supérieure de la boucle est pourvu d'une partie saillante de surface supérieure de boucle, dans lequel une pluralité de rainures avec différentes profondeurs sont formées dans une face de la partie saillante de surface supérieure de boucle agencée sur la surface supérieure de la boucle, et une pente inverse est prévue sur l'autre face de la partie saillante de surface supérieure de boucle agencée sur la surface supérieure de la boucle, dans lequel l'extrémité arrière de la boucle qui est pourvue de la pluralité de rainures avec différentes profondeurs est en butée contre la bague d'étanchéité annulaire, dans lequel la surface intérieure du mécanisme de verrouillage extérieur est pourvue d'une pluralité de bagues convexes avec différentes hauteurs, et chacune des rainures avec différentes profondeurs sur la surface supérieure de la boucle interagit respectivement avec chacune des bagues convexes avec différentes hauteurs sur la surface intérieure du mécanisme de verrouillage extérieur.

6. Bistouri à haute fréquence absorbant la fumée à fonction d'éclairage et apte à verrouiller la longueur télescopique selon la revendication 1, dans lequel le bistouri à haute fréquence absorbant la fumée à fonction d'éclairage et apte à verrouiller la longueur télescopique comprend en outre un siège de fixation du ressort conducteur du tube absorbant la fumée et un ressort conducteur à haute fréquence, dans lequel une extrémité du tube absorbant la fumée est reliée de manière interne au siège de fixation du ressort conducteur du tube absorbant la fumée, dans lequel une extrémité du ressort conducteur à haute fréquence est reliée de manière interne au siège de fixation du ressort conducteur du tube absorbant la fumée, et l'autre extrémité du ressort conducteur à haute fréquence est reliée de manière interne à une rainure en cuivre conductrice à haute fréquence (54).

7. Bistouri à haute fréquence absorbant la fumée à fonction d'éclairage et apte à verrouiller la longueur télescopique selon la revendication 1, dans lequel le bistouri à haute fréquence absorbant la fumée à fonction d'éclairage et apte à verrouiller la longueur télescopique comprend en outre un manchon antidérapant (84), et le corps principal de bistouri est recouvert par le manchon antidérapant.

8. Bistouri à haute fréquence absorbant la fumée à fonction d'éclairage et apte à verrouiller la longueur télescopique selon la revendication 1, dans lequel le bistouri à haute fréquence absorbant la fumée à fonction d'éclairage et apte à verrouiller la longueur télescopique comprend en outre un joint courbé (81), un tuyau (82) et un fil transporteur de courant (83), dans lequel un côté du joint courbé est relié de manière simultanée à la coque supérieure de bistouri et au corps principal de bistouri dans un mode manchonné, et l'autre côté du joint courbé est relié de manière interne au tuyau, dans lequel le fil transporteur de courant s'étend à l'intérieur de la première chambre de réception formée entre la coque supérieure de bistouri et le corps principal de bistouri à travers le joint courbé.

9. Bistouri à haute fréquence absorbant la fumée à fonction d'éclairage et apte à verrouiller la longueur télescopique selon la revendication 1, dans lequel le bistouri à haute fréquence absorbant la fumée à fonction d'éclairage et apte à verrouiller la longueur télescopique comprend en outre un bouton d'éclairage (14), un bouton de coagulation électrique et un bouton de découpage électrique, dans lequel le bouton d'éclairage est utilisé pour entrer le premier signal de commande, le bouton de coagulation électrique est utilisé pour entrer le deuxième signal de commande, et le bouton de découpage électrique est adapté à être utilisé pour entrer le troisième signal de commande, dans lequel la coque supérieure de bistouri est pourvue d'un premier trou de positionnement, d'un deuxième trou de positionnement et d'un troisième trou de positionnement, dans lequel le bouton d'éclairage, le bouton de coagulation électrique et le bouton de découpage électrique sont respectivement intégrés dans le premier trou de positionnement, le deuxième trou de positionnement et le troisième trou de positionnement.
